# EUROPÄISCHE PATENTSCHRIFT

(11) **EP 3 990 895 B1**
(45) Veröffentlichungstag und Bekanntmachung des Hinweises auf die Patenterteilung: **21.02.2024**
(21) Anmeldenummer: 20733373.3
(22) Anmeldetag: 08.06.2020
(51) Int. Cl.: G01N 21/77, G01N 21/64, G01N 21/78

(54) **SENSORMODUL ZUR MULTIPARAMETRISCHEN ANALYSE EINES MEDIUMS**
SENSOR MODULE FOR MULTIPARAMETRICALLY ANALYSING A MEDIUM
MODULE DE CAPTEURS POUR L'ANALYSE MULTIPARAMÉTRIQUE D'UN MILIEU

(30) Priorität: 25.06.2019 DE 102019117045
(43) Veröffentlichungstag der Anmeldung: 04.05.2022
(73) Patentinhaber: Sentronic GmbH Gesellschaft für optische Meßsysteme, 01217 Dresden (DE)
(72) Erfinder: LAU, Matthias, 01217 Dresden (DE)
(74) Vertreter: Kailuweit & Uhlemann Patentanwälte Partnerschaft mbB
(86) Internationale Anmeldenummer: PCT/EP2020/065837
(87) Internationale Veröffentlichungsnummer: WO 2020/259996

(56) Entgegenhaltungen:
- US-A1- 2014 001 058
- KRUJATZ F ET AL: "Exploiting the Potential of OLED-Based Photo-Organic Sensors for Biotechnological Applications", CHEMICAL SCIENCES JOURNAL, Bd. 7, Nr. 3, 28. Juli 2016 (2016-07-28), XP055398420, DOI: 10.4172/2150-3494.1000134 in der Anmeldung erwähnt
- JOSEPH SHINAR ET AL: "TOPICAL REVIEW; Organic light-emitting devices (OLEDs) and OLED-based chemical and biological sensors: an overview", JOURNAL OF PHYSICS D: APPLIED PHYSICS, INSTITUTE OF PHYSICS PUBLISHING LTD, GB, Bd. 41, Nr. 13, 7. Juli 2008 (2008-07-07), Seite 133001, XP020133400, ISSN: 0022-3727
- COYLE S ET AL: "BIOTEX-Biosensing Textiles for Personalised Healthcare Management", IEEE TRANSACTIONS ON INFORMATION TECHNOLOGY IN BIOMEDICINE, IEEE SERVICE CENTER, LOS ALAMITOS, CA, US, Bd. 14, Nr. 2, 1. März 2010 (2010-03-01), Seiten 364-370, XP011345679, ISSN: 1089-7771, DOI: 10.1109/TITB.2009.2038484

## Beschreibung

Die Erfindung betrifft ein Sensormodul zur multiparametrischen Analyse eines Mediums aufweisend mindestens einen organischen Lichtemitter zur Aussendung eines photonischen Signals und mindestens eine Vorrichtung zur Detektion photonischer Signale, wobei der mindestens eine organische Lichtemitter und die mindestens eine Vorrichtung zur Detektion photonischer Signale monolithisch auf einem Halbleitersubstrat oder in einem Halbleitersubstrat ausgebildet sind, und des Weiteren aufweisend mindestens ein Funktionsschichtsystem, das den mindestens einen organischen Lichtemitter und/oder die mindestens eine Vorrichtung zur Detektion photonischer Signale zumindest teilweise überdeckt und in Kontakt mit dem Medium ist, wobei die Funktionsschicht so ausgebildet ist, dass sie mindestens einen aktiven Bereich aufweist, der mindestens eine Eigenschaft besitzt, die durch mindestens eine Eigenschaft des Messmediums beeinflussbar ist, sowie die Verwendung des Sensormoduls zur multiparametrischen Analyse eines Mediums.

Um ein Medium multiparametrisch, also anhand mehrerer, quasi-simultan zu messender physikalischer, chemischer, biochemischer und/oder biologischer Parameter, zu analysieren, werden häufig Kombinationen von Einzelsensoren für jeweils einen der Parameter eingesetzt, deren Miniaturisierungspotenzial sehr begrenzt ist Weitere Nachteile sind der hohe Energieverbrauch, der große Aufwand zur Verbesserung des Signal-Rauschverhältnisses und zur Gewährleistung der elektromagnetischen Verträglichkeit. Insbesondere auch aufgrund der hohen Herstellungskosten können diese Einzelsensor-Kombinationen ökologisch und wirtschaftlich nicht sinnvoll als preisgünstiges Einwegmodul ausgelegt werden. In der aktuellen Praxis führt dies zur Trennung von Messwertaufnehmer und Transmitter/Verarbeitungseinheit, wobei häufig nur der Messwertaufnehmer, manchmal auch mit Transmitter/Vorverarbeitung, als Einwegartikel ausgelegt ist. Nach einem Austausch muss dann kundenseitig eine Justage/Kalibrierung erfolgen, was die Nutzungskosten erhöht, teilweise zusätzliche Prüftechnik sowie ausreichende Qualifizierungen benötigt und die Fehleranfälligkeit steigert sowie Kreuzkontaminationen, z. B. in kritischen Anwendungen der pharmazeutischen Industrie, bedingen kann.

Speziell für biologische, biochemische oder chemische Messaufgaben kann beispielsweise die gleichzeitige Bestimmung von pH-Wert, Leitfähigkeit, Temperatur, Konzentration interessant sein.

Aus dem Stand der Technik, bspw. [Krujatz2016], sind OLED-basierte organische Photosensoren für biotechnologische Anwendungen bekannt. Derartige Sensoren weisen organische Leuchtdioden (OLED) als Lichtquelle sowie Photodetektoren auf und werden als Photolumineszenzsensor, Biosensor oder Absorptions-/Transmissionssensor bspw. zur Bestimmung von gelöstem Sauerstoff in wässrigen Lösungen, der Sauerstoffsättigung im Blut oder zur enzymatischen Detektion von Glukose eingesetzt. Die OLEDs sind auf Substraten, wie Folien, Glas oder Silizium, aufgebracht. Weiterhin sind OLED-basierte Sensorarrays zur gleichzeitigen Detektion unterschiedlicher Parameter des Analyten, wie bspw. von gelöstem Sauerstoff, Lactat, Glukose u.a., bekannt.

Die DE 10 2007 056 275 B3 offenbart einen Chip zum Analysieren eines Mediums, wobei auf einem aktiven CMOS-Substrat organische Halbleiter zur Beleuchtung des Mediums und Photodetektoren in einer Matrix-Anordnung monolithisch integriert sind. Zur Signalverarbeitung können weitere aktive elektronische Elemente im Substrat integriert sein.

In der DE 10 2006 030 541 A1 wird eine optische Anordnung bspw. zur Erfassung physiologischer Parameter von Lebewesen oder zur visuellen Anzeige von Informationen beschrieben. Dabei sind auf einem gemeinsamen Substrat bevorzugt mindestens eine organische Leuchtdiode und mindestens eine Photodiode und/oder eine CMOS-Photodiode angeordnet oder daran ausgebildet und mit einer elektronischen Auswerte- und Steuereinheit verbunden. Die Anordnung kann weitere aktive und passive elektronische/elektische Bauelemente zur Verstärkung, Speicherung und/oder Ansteuerung der einzelnen Elemente aufweisen.

Die WO 2011/048472 A1 zeigt ein optisches Sensorsystem zur Immundiagnostik mit einem Schichtaufbau und einer OLED als Lichtquelle, bei dem ein fluoreszierendes oder phosphoreszierendes Markerelement an einen Antikörper bindet.

Die EP 1 672 356 B1 offenbart einen optischen Sensor zur Einmalnutzung, bei dem eine Lichtquelle und eine Photodiode auf einem Substrat angeordnet und durch eine Trennschicht von einer Funktionsschicht, die in Abhängigkeit von einem Analyten ein optisches Signal aussendet, getrennt sind.

Aus der EP 2 988 807 B1 ist eine Sensorvorrichtung mit einer OLED als Lichtquelle und einem akustischen Sensor bekannt.

Aus DE 10 2014 010 116 B4 ist ein MEMS-Sensor zur Druckmessung in CMOS-Technologie bekannt, dessen Vorderseite mit dem zu messenden Medium in Kontakt steht und einen zuverlässigen Schutz des Sensors vor Korrosion bietet.

DE 10 2016 220 086 A1 beschreibt ein mikrostrukturiertes organisches Sensorbauelement aus einer Vielzahl von Sensorsegmenten, die auf CMOS-Substraten angeordnet sind. Auf dem Substrat kann zusätzlich mindestens eine Leuchtdiode mit jeweils einem oder mehreren Emittern angeordnet sein.

Die EP 2 955 759 B1 offenbart ein Herstellungsverfahren für ein Halbleiterbauelement mit einem Photodetektor auf einem Substrat, das eine dielektrische Schicht mit eingebetteter Verdrahtung aufweist.

Shinar, J., et al.: "Organic light-emitting devices (OLEDS) and OLED-based chemical and biological sensors: an overview", JOURNAL OF PHYSICS D: APPLIED PHYSICS 41 (2016), 13, 133001, gibt einen Überblick über OLED-basierte chemische und biologische Sensoren unter Nutzung von Photolumineszens. Es wird auch die Möglichkeit zur strukturellen Integration von OLEDs und einem Photodetektor auf einem Glas- oder Plastiksubstrat offenbart.

Die US 2014/0001058 A1 beschreibt eine Testvorrichtung, bei der in oder auf einem flexiblen Substrat monolithisch ein Behältnis für die zu testende biologische Probe angeordnet ist Durch eine optische oder elektrische Anregung oder eine chemische Reaktion werden Ausgangssignale der Probe erzeugt, die in einem elektrischen Schaltkreis ausgewertet werden. Die Testvorrichtung kann dazu eine Lichtquelle aufweisen, um den Probenbehälter zu beleuchten und ein entsprechendes Ausgangssignal zu erzeugen, welches mit einem Photodetektor gemessen werden kann.

Coyle, S., et al.: "BIOTEX - Biosensing Textiles for Personalised Healthcare Management", IEEE TRANSACTIONS ON INFORMATION TECHNOLOGY IN BIOMEDICINE 14 (2010), 2, 364 befasst sich mit textilen Sensoren, mit denen Schweiß (pH-Wert, Natriumkonzentration, elektrische Leitfähigkeit), die Sauerstoffsättigung des Bluts, die Herztätigkeit (EKG) und die Atemfrequenz zu Gesundheitsmanagementzwecken analysiert werden können. Zur Analyse der Sauerstoffsättigung des Bluts und des pH-Werts des Schweißes werden photonische Messprinzipien angewendet, für die anderen Parameter nicht-photonische Messprinzipien. Als Substrate für die Sensoren werden Textilien oder Kunststoffe verwendet.

Aus dem Stand der Technik sind keine multiparametrischen Sensormodule bekannt, die photonische und nicht-photonische Messprinzipien mit parametersensitiven Beschichtungen auf einem Substrat kombinieren.

Aufgabe der vorliegenden Erfindung ist es daher, eine Vorrichtung anzugeben, mittels derer mehrere Eigenschaften eines Mediums über weite Parameterbereiche hinweg mit einem Sensormodul erfasst werden können, wobei die für den jeweiligen Parameter mindestens z. B. bezüglich der Genauigkeit, der Langzeitstabilität, der Auflösung, der Reproduzierbarkeit, dem Energieverbrauch, den Herstellkosten, dem notwendigen Platzbedarf geeignetste Methode Anwendung finden kann.

Die Aufgabe wird gelöst durch ein Sensormodul mit den Merkmalen des Anspruchs 1. Weiterbildungen der Erfindung sind in Unteransprüchen angegeben.

Die erfindungsgemäße Lösung basiert darauf, dass das Sensormodul photonische und nicht-photonische Messprinzipien auf vorteilhafte Weise auf demselben Halbleitersubstrat miteinander kombiniert.

Unter "photonischen Messprinzipien" wird im Sinne dieser Anmeldung die Anwendung optischer Verfahren und Technologien zur Detektion photonischer Signale verstanden, wobei photonische Signale prinzipiell Photonen des gesamten elektromagnetischen Spektrums umfassen können, die mittels der Messtechnik in elektrische Signale umgewandelt werden können. Erfindungsgemäß umfassen die photonischen Signale insbesondere Photonen mit Wellenlängen im Bereich des sichtbaren Lichts oder im Bereich des nahen Infrarot-Lichtes.

Unter "nicht-photonischen Messprinzipien" werden im Sinne dieser Anmeldung Messverfahren verstanden, die nicht auf der Detektion von Photonen als Informationsträger beruhen.

Das Sensormodul weist mindestens einen organischen Lichtemitter, insbesondere eine organische Leuchtdiode (OLED), und mindestens eine Vorrichtung zur Detektion photonischer Signale (Photodetektor, PD) auf, die monolithisch auf oder in einem Halbleitersubstrat ausgebildet sind. Im Sinne der Erfindung ist unter "monolithisch" zu verstehen, dass der organische Lichtemitter und die Vorrichtung zur Detektion photonischer Signale eine untrennbare Einheit mit dem Halbleitersubstrat bilden. Beispielsweise bilden sich in verschiedenen Halbleiterprozessen, z. B. dem CMOS-Prozess, bauteilinhärente Photodioden, die als Photodetektoren dienen können, an pn-Grenzflächen aus.

Das Sensormodul weist des Weiteren mindestens ein Funktionsschichtsystem auf, das den mindestens einen organischen Lichtemitter und/oder den mindestens einen PD zumindest teilweise überdeckt und mittelbar oder unmittelbar in Kontakt mit dem zu analysierenden Medium ist. Das Funktionsschichtsystem ist so ausgebildet, dass es mindestens einen aktiven Bereich aufweist, der mindestens eine Eigenschaft besitzt, die durch mindestens eine Eigenschaft des Mediums beeinflussbar ist. Dies ist so zu verstehen, dass der mindestens eine aktive Bereich des Funktionsschichtsystems mindestens eine sensorisch aktive Komponente enthält, deren Interaktion mit dem zu analysierenden Medium die Antwort der sensorisch aktiven Komponente auf das von dem mindestens einen organischen Lichtemitter emittierte erste photonische Signal beeinflusst. Die sensorisch aktive Komponente emittiert ein, mit dem ersten photonischen Signal in Beziehung stehendes, zweites photonisches Signal. Das zweite photonische Signal kann z. B. eine (Teil-)Reflexion und/oder (Teil-)Absorption und/oder (Teil-)Streuung/-Rückstreuung des ersten photonischen Signals sein und gegenüber dem ersten photonischen Signal intensitätsmoduliert sein, oder das zweite photonische Signal kann gegenüber dem ersten photonischen Signal z. B. durch Fluoreszenz auch wellenlängenmoduliert sein. Die Detektion dieser photonischen Antwort, die Informationen über eine Eigenschaft des zu analysierenden Mediums enthält, erfolgt mittels des mindestens einen PD. Die sensorisch aktive Komponente des Funktionsschichtsystems kann beispielsweise eine funktionelle Gruppe, ein Farbstoff, ein Enzym, ein Protein, ein Antikörper, eine Nukleinsäure, ein Virus oder ein Edelmetallclustersein. Die sensorisch aktive Komponente kann beispielsweise auch ein Polymer sein, dessen Quellverhalten vom pH-Wert oder von der Temperatur des Mediums abhängig ist.

Der Begriff "Eigenschaft" umfasst im Sinne dieser Anmeldung auch Eigenschaftsänderungen; insbesondere kann das zweite photonische Signal Informationen über Absolut- und/oder Relativwerte einer Eigenschaft des Mediums enthalten.

Die Beeinflussung der photonischen Antwort durch Interaktion mit dem Medium kann beispielsweise per Lumineszenzspektroskopie (z. B. Fluoreszenzspektroskopie), Spektralphotometrie (z. B. Absorptionsmessung, Reflexionsmessung), Farbmessung (z. B. ratiometrisch, photometrisch, Farbmetrik/Farbänderung), Plasmonenresonanz (SPR) und/oder nicht-dispersiver Infrarotmessverfahren (NDIR) detektiert werden.

Erfindungsgemäß ist des Weiteren das Halbleitersubstrat so ausgebildet, dass zumindest eine zweite, vorzugsweise von der photonisch ermittelten Eigenschaft des Mediums verschiedene, Eigenschaft mittels eines nicht-photonischen Messprinzips ermittelbar ist, oder das Sensormodul weist mindestens ein Bauelement zur Bestimmung zumindest einer zweiten Eigenschaft auf, wobei das Bauelement auf dem Halbleitersubstrat angeordnet ist.

Vorteilhaft bietet das Sensormodul eine hochintegrierte Kombination verschiedener Sensortechnologien. Das erfindungsgemäße Sensormodul ist für eine preiswerte Massenfertigung unter Nutzung bewährter Halbleiterfertigungstechnologien geeignet. Dadurch kann für jeden zu messenden Parameter die mindestens z. B. bezüglich der Genauigkeit, der Langzeitstabilität, der Auflösung, der Reproduzierbarkeit, dem Energieverbrauch, den Herstellkosten, dem notwendigen Platzbedarf geeignetste Methode Anwendung finden und dennoch z. B. Energie, Baugröße und Herstellkosten gegenüber herkömmlichen Lösungen eingespart werden.

Aufgrund der Größen-, Energieverbrauchs- und Kostenminimierung ist es möglich, das erfindungsgemäße Sensormodul als Einwegartikel auszulegen, was vorteilhaft eine Werkskalibrierung (factory calibration) aller Parameter gestattet. Es wird eine effektive Selbstüberwachung und/oder Kompensation bzw. Referenzierung ermöglicht.

Des Weiteren bietet das erfindungsgemäße Sensormodul die Möglichkeit, den Messort von photonischem und nicht-photonischem Messprinzip auf einfache Weise vorteilhaft wählen zu können.

Das Funktionsschichtsystem wirkt zugleich als Abschirmung von organischem Lichtemitter und PD oder Halbleiterchip gegenüber der Umgebung.

In einer Ausführungsform der erfindungsgemäßen Vorrichtung ist das Halbleitersubstrat als CMOS ausgebildet. Mittels der OLED-on-Si-CMOS-Technologie kann die integrierte CMOS-Schaltungstechnik durch einen stabilen, effizienten Lichtemitter ergänzt werden, wobei die OLED monolithisch über der CMOS-Backplane angeordnet werden kann.

In Ausführungsformen der erfindungsgemäßen Vorrichtung umfasst das Halbleitersubstrat mindestens eine Vorrichtung zur Durchführung einer elektrochemischen Messung oder mindestens eine Vorrichtung zur Durchführung einer Temperaturmessung oder mindestens eine Vorrichtung zur Durchführung einer Impedanzmessung oder mindestens eine Vorrichtung zur Durchführung einer Magnetfeldmessung oder mindestens eine Vorrichtung zur Durchführung einer Rückstreumessung oder mindestens eine Vorrichtung zur Durchführung einer Durchflussmessung oder mindestens eine Vorrichtung zur Durchführung einer Strömungsgeschwindigkeitsmessung oder mindestens eine Vorrichtung zur Durchführung einer Wärmestrommessung oder mindestens eine Vorrichtung zur Durchführung einer Druckmessung oder eine Kombination der vorbenannten. Die Vorrichtungen können vorteilhaft monolithisch in das Halbleitersubstrat integriert sein. Es kann auch vorteilhaft sein, dass die Vorrichtungen in das Funktionsschichtsystem integriert und/oder auf dem Funktionsschichtsystem angeordnet sind.

Die mindestens eine Vorrichtung zur Durchführung einer elektrochemischen Messung umfasst bevorzugt eine Electrode-on-CMOS- oder ISFET- oder ChemFET- oder ENFET- oder pH-FET oder Solid-state-electrolyte-Struktur oder eine Kombination aus den vorbenannten.

Die mindestens eine Vorrichtung zur Durchführung einer Temperaturmessung umfasst bevorzugt einen R-on-CMOS- oder einen Thermoelement- oder einen Halbleiter-Sensor oder eine Kombination aus den vorbenannten.

Die mindestens eine Vorrichtung zur Durchführung einer Impedanzmessung umfasst bevorzugt eine interdigitale Elektrodenstruktur.

Die mindestens eine Vorrichtung zur Durchführung einer Magnetfeldmessung umfasst bevorzugt einen Hallsensor.

Die mindestens eine Vorrichtung zur Durchführung einer Strömungsgeschwindigkeitsmessung umfasst bevorzugt das Prinzip eines Hitzdrahtanemometers.

Die mindestens eine Vorrichtung zur Durchführung einer Wärmestrommessung umfasst bevorzugt einen Thermosäulensensor.

Die mindestens eine Vorrichtung zur Durchführung einer Druckmessung umfasst bevorzugt einen Dünnfilm- oder Dickfilm- oder piezoresistiven oder MEMS-Sensor oder eine Kombination aus den vorbenannten.

In einer weiteren bevorzugten Ausführungsform weist das erfindungsgemäße Sensormodul mehrere organische Lichtemitter auf, die räumlich voneinander getrennt an definierbaren Positionen auf oder im Halbleitersubstrat anordenbar sind, und die jeweils ein erstes photonisches Signal aussenden, wobei die Wellenlängen der ersten photonischen Signale unterschiedlicher organischer Lichtemitter voneinander verschieden oder gleich sein können. Die mehreren organischen Lichtemitter können in Segmenten oder Arrays angeordnet sein.

In einer weiteren bevorzugten Ausführungsform weist das erfindungsgemäße Sensormodul mehrere Vorrichtungen zur Detektion photonischer Signale auf, die räumlich voneinander getrennt an definierbaren Positionen auf oder im Halbleitersubstrat anordenbar sind. Dabei können die mehreren Vorrichtungen zur Detektion photonischer Signale so ausgebildet sein, dass sie voneinander verschiedene spektrale Sensitivitätsbereiche aufweisen, so dass voneinander verschiedene Vorrichtungen photonische Signale unterschiedlicher Wellenlängen detektieren können. Die mehreren Vorrichtungen zur Detektion photonischer Signale können aber auch so ausgebildet sein, dass sie die gleichen oder sich überlappende Sensitivitätsbereiche aufweisen, wobei eine Zuordnung des jeweils detektierten zweiten photonischen Signals zum Ort seiner Aussendung über unterscheidbare Überlappungen der numerische Aperturen erfolgt. Die mehreren Vorrichtungen zur Detektion photonischer Signale können ebenfalls in Segmenten oder Arrays angeordnet sein.

Vorteilhaft bietet die Ausgestaltung des erfindungsgemäßen Sensormoduls mit mehreren organischen Lichtemittern und mehreren Vorrichtungen zur Detektion photonischer Signale die Möglichkeit zur ortsaufgelösten Analyse des Mediums., und dies besonders vorteilhaft auch bei einem kleinen aktiven Bereich des Funktionsschichtsystems. Im Sinne dieser Erfindung wird unter "ortsaufgelöst" die Zuordnung detektierter Signale zu einem Messort bzw. Entstehungsort verstanden.

Verschiedene Konzepte von Ortsauflösung in Bezug auf das erfindungsgemäße Sensormodul werden im Folgenden näher erläutert.

Zum einen kann ein aktiver Bereich des Funktionsschichtsystems mehrere sensorisch aktive Komponenten, z. B. Farbstoffe, aufweisen, die mittels voneinander verschiedener organischer Lichtemitter, die erste photonische Signale mit voneinander verschiedenen Wellenlängen aussenden, angeregt werden und zweite photonische Signale mit voneinander verschiedenen Wellenlängen aussenden, die von den entsprechenden, voneinander verschiedenen PD mit voneinander verschiedenen spektralen Sensitivitätsbereichen detektiert werden.

Zum anderen kann es aber auch sein, dass die mehreren sensorisch aktiven Komponenten eines aktiven Bereichs des Funktionsschichtsystems zwar unterschiedlich angeregt werden, aber zweite photonische Signale im gleichen Sensitivitätsbereich aussenden In diesem Fall ist eine Zuordnung der mittels voneinander verschiedener PD detektierten zweiten photonischen Signale zum Ort ihrer Aussendung, insbesondere also zu einer der sensorisch aktiven Komponenten, über die numerische Apertur des optischen Systems aus Lichtemitter - sensorisch aktiver Komponente - PD vornehmbar.

Des Weiteren kann im vorliegenden Fall auch eine zeitversetzte Anregung der sensorisch aktiven Komponenten erfolgen, oder eine Mischung/Überlagerung der zweiten photonischen Signale detektiert werden.

In einer weiteren bevorzugten Ausführungsform des erfindungsgemäßen Sensormoduls ist mindestens eine Vorrichtung zur Detektion photonischer Signale unmittelbar unterhalb mindestens eines organischen Lichtemitters angeordnet. Vorteilhaft kann in dieser Konfiguration direkt das emittierte erste photonische Signal zur Referenzierung z. B. von Intensitäts- und/oder Phasenschwankungen vermessen werden.

Zum Zwecke der Referenzierung des Sensormoduls kann mindestens eine Kombination von mit Hilfe zweier zweiter photonischer Signale oder zweier nicht-photonischer Signale oder eines zweiten photonischen und eines nicht-photonischen Signals generierten Messwerten dienen. Besonders vorteilhaft ermöglicht die Ortszuweisung von organischem Lichtemitter und PD auf dem erfindungsgemäßen Sensormodul eine Referenzierung direkt an der zu referenzierenden Position.

In einer weiteren bevorzugten Ausführungsform des erfindungsgemäßen Sensormoduls weist das Funktionsschichtsystem mehr als einen aktiven Bereich auf, wobei die aktiven Bereiche räumlich voneinander getrennt angeordnet sind. Die räumliche Trennung umfasst auch eine Anordnung mehrerer aktiver Bereiche übereinander, also im gleichen optischen Pfad zwischen organischem Lichtemitter und Vorrichtung zur Detektion photonischer Signale.

Die aktiven Bereiche können zum einen die gleichen sensorisch aktiven Komponenten enthalten und für dieselbe Eigenschaft des Mediums sensitiv sein. Aufgrund der räumlichen Trennung der aktiven Bereiche ist in dieser Konfiguration eine ortsaufgelöste Analyse des Mediums hinsichtlich einer bestimmten Eigenschaft möglich.

Zum anderen können die aktiven Bereiche so ausgebildet sein, dass sie für voneinander verschiedene Eigenschaften sensitiv sind. Dies kann beispielsweise über unterschiedliche sensorisch aktive Komponenten realisiert werden, oder dadurch, dass mindestens einer der mehreren aktiven Bereiche eine definierte Beschichtung aufweist. Vorteilhaft ermöglicht das erfindungsgemäße Sensormodul damit die multiparametrische Analyse des Mediums auf besonders kompakte, einfache und miniaturisierte Weise.

Die mehreren aktiven Bereiche des Funktionsschichtsystems können auch voneinander verschiedene spektrale Sensitivitäten aufweisen, wobei sich die Spektralbereiche, in denen die sensorisch aktiven Komponenten unterschiedlicher aktiver Bereiche photonisch anregbar sind, überlappen können. Diese Ausführung ist vorteilhaft mit der erfindungsgemäßen Ausführung des Sensormoduls mit mehreren organischen Lichtemittern und mehreren Vorrichtungen zur Detektion photonischer Signale kombinierbar.

Eine beispielhafte vorteilhafte Ausführungsform umfasst die Anregung eines unteren aktiven Bereichs von mehreren untereinander angeordneten aktiven Bereichen des Funktionsschichtsystems mittels eines ersten photonischen Signals, wobei dieser untere aktive Bereich ein zweites photonisches Signal (welches im Allgemeinen einen anderen Wellenlängenbereich umfasst) an einen im optischen Pfad darüber angeordneten, weiteren aktiven Bereich aussendet, wodurch in diesem weiteren aktiven Bereich sensorisch aktive Komponenten angeregt werden und ein drittes photonisches Signal, moduliert durch die von mindestens einer Eigenschaft des umgebenden Mediums hervorgerufenen Eigenschaftsänderungen dieses weiteren aktiven Bereiches, ausgesendet wird, wobei für dieses dritte photonische Signal mindestens die zwischen dem Halbleitersubstrat und diesem weiteren aktiven Bereich angeordneten Bereiche/Schichten des Funktionsschichtsystems transparent sind, so dass dieses Signal, in diesem Fall als drittes photonisches Signal bezeichnet, durch mindestens eine Photodiode empfangen werden kann.

Zusammenfassend bietet das erfindungsgemäße Sensormodul die Möglichkeit zur ortsaufgelösten Analyse in zweierlei Aspekten:
- Zum einen in Bezug auf das Medium, das heißt, das detektierte zweite photonische Signal und/oder das nicht-photonische Signal können/kann dem Messort im Medium zugeordnet werden und beschreibt die Eigenschaft des Mediums an diesem Ort;
- Zum anderen in der internen Zuweisung von ausgesendetem ersten photonischen Signal und detektiertem zweiten photonischen Signal mittels Ausnutzung der numerischen Apertur (NA).

In einer weiteren Ausführungsform umfasst das Funktionsschichtsystem des erfindungsgemäßen Sensormoduls mindestens eine Funktionsschicht, die auf einem Funktionsschichtträger angeordnet ist. Das Funktionsschichtsystem kann aus mehreren Schichten aufgebaut sein, von denen nur eine oder mehrere aktive Bereiche aufweisen.

Im Allgemeinen ist der Funktionsschichtträger für die Wellenlänge des ersten und des zweiten photonischen Signals transparent. Insbesondere kann der Funktionsschichtträger als optische Linse oder als optisches Linsenarray oder als optischer Filter oder als optisches Gitter oder als Kombination aus den vorbenannten ausgebildet sein. Der Funktionsschichtträger kann direkt auf dem Halbleitersubstrat oder zu diesem beabstandet angeordnet sein. Der Funktionsschichtträger kann auch als Teil eines das Halbleitersubstrat aufnehmenden Gehäuses ausgebildet sein oder durch direkte Fügeverfahren (z.B. Kleben, anodisches Bonden, Adhäsionsverbinden, Eingießen) das Halbleitersubstrat (inklusive möglicher Aufbauten) kapseln/verschließen. Dies ermöglicht vorteilhaft eine effektive, z. B. nicht-parasitär lumineszierende Modulverkapselung.

Das erfindungsgemäße Sensormodul umfasst zumindest eine der in den zwei folgenden Absätzen beschriebenen Vorrichtungen.

In einer Ausführungsform weist das erfindungsgemäße Sensormodul mindestens eine Vorrichtung zum Speichern von Daten, z. B. Messdaten, Programmcode, Logbuch-Daten, Historie-Daten, und/oder zum Auswerten und Beeinflussen von Daten, z. B. zum Kompensieren, und/oder zur Übertragung von Daten, z. B. Messdaten, Auswertedaten, Statusinformationen, Datenlogger-Daten, und/oder zur Kommunikation auf. Übertragung und Kommunikation können digital und/oder analog sowie kabelgebunden oder kabellos erfolgen. Das Sensormodul kann auch autonom arbeiten, und es können nach Abschluss der Messung die Messdaten ausgelesen werden.

Zusätzlich oder alternativ weist das erfindungsgemäße Sensormodul mindestens eine Vorrichtung zur Ansteuerung und/oder zur Modulation/Demodulation des mindestens einen organischen Lichtemitters und/oder der mindestens einen Vorrichtung zur Detektion photonischer Signale auf.

Erfindungsgemäß sind die vorbenannten Vorrichtungen monolithisch im Halbleitersubstrat ausgebildet. In einer weiteren Ausführungsform weist das erfindungsgemäße Sensormodul mindestens eine Vorrichtung zur Bereitstellung der für den Betrieb des Sensormoduls aufzuwendenden elektrischen Energie auf. Dabei kann es sich z. B. um eine Batterie oder Superkapazität handeln. Die Vorrichtung kann auch dazu geeignet sein, andere Energieformen in elektrische Energie umzuwandeln, z. B. potentielle Energie des Schalls, kinetische Energie oder thermische Energie, wie allgemein als Energy Harvesting bekannt. Weiterhin kann die Energie drahtlos übertragen werden, z.B. durch Standards wie NFC oder Ql, oder auch durch Feldkopplung induziert werden. Vorteilhaft kann das Sensormodul dadurch autonom arbeiten.

In einer weiteren Ausführungsform weist das erfindungsgemäße Sensormodul mindestens eine aktorische Komponente auf, z. B. eine Heizung zur Temperierung, insbesondere thermischen Stabilisierung, des Sensormoduls oder zur Aktivierung von Enzymen im Funktionsschichtsystem, oder aktorische Komponenten zur Steuerung von Mikrofluidik. Vorteilhaft ist die Aktorik bei dem erfindungsgemäßen Sensormodul energieeffizient und reaktionsbeschleunigt auf kleinsten Raum integrierbar. Aufgrund der kompakten Bauform kann die thermische Stabilisierung des Sensormoduls energieeffizient und mit kurzen Regelzeiten ausgeführt werden.

Des Weiteren lassen sich mehrere erfindungsgemäße Sensormodule mit einem Basismodul, z. B. in einer kubischen Anordnung, verbinden, und innerhalb des Basismoduls beispielsweise eine gemeinsame Energieversorgung, Datenverarbeitung, Datenspeicherung oder Kommunikation benutzen, wobei die Sensormodule separat austauschbar oder modular montiert werden können. Die geringe und kompakte Bauform der erfindungsgemäßen Sensormodule ermöglicht schnellere Reaktionszeiten, schnellere Ansprechzeiten und kürzere Einschwingzeiten der Sensormodulkombination.

Das erfindungsgemäße Sensormodul wird bevorzugt in einem Lab-on-a-chip-System verwendet. Dazu kann Mikrofluidik direkt oder indirekt an das Funktionsschichtsystem angekoppelt werden, oder die Mikrofluidik kann Strukturteil des Halbleitersubstrates oder des Funktionsschichtsystems sein.

Das erfindungsgemäße Sensormodul kann vorteilhaft in einfacher Weise sterilisierbar bzw. desinfizierbar ausgeführt werden. Dadurch ergeben sich weitere bevorzugte Verwendungen.

Des Weiteren bevorzugt ist die Verwendung des erfindungsgemäßen Sensormoduls zur Fermentationsüberwachung, insbesondere in einem Einweg-Fermentationsbeutel. Diese werden bereits werkseitig vom Hersteller zumeist mit Gamma-Strahlung sterilisiert. Das nachträgliche Einführen von Messtechnik zum Erfassen der Steuerparameter ist umständlich und kann zur nachträglichen Kontamination des Einweg-Fermentationsbeutels führen. Deshalb müssen beispielsweise in der pharmazeutischen Industrie umfangreiche Prozeduren zur Überprüfung der Bereinigung abgearbeitet werden, die teuer, zeitaufwendig sind und wiederum die Gefahr von Kreuzkontamination bergen. Aufgrund der Sterilisierbarkeit des erfindungsgemäßen Sensormoduls kann dieses bereits vor der Sterilisation in den Einweg-Fermentationsbeutel eingebracht werden. Das erfindungsgemäße Sensormodul ist preiswert und biokompatibel, sowie einfach und ohne Umweltschäden entsorg- bzw. recycelbar, so dass es vorteilhaft als Einwegartikel verwendbar ist. Neben der Gamma-Sterilisierung sind weitere Desinfektions- und Sterilisierungsmöglichkeiten gegeben, z. B. mittels Desinfektionsmittel, durch Autoklavierung (steam-sterilizable), durch Plasmasterilisation, durch UV-Strahlung.

Des Weiteren bevorzugt ist die Verwendung des erfindungsgemäßen Sensormoduls zur Überwachung von Vitalparametern. Dies beinhaltet z. B. die Messung der CO₂- und/oder O₂-Konzentration in der Atemluft, Glukose- und/oder Laktatkonzentration im Speichel. Eine besonders vorteilhafte Parameterkombination kann durch transkutane Messungen des CO₂- und/oder O₂-Partialdrucks mittels eines photonischen Messprinzips sowie Temperatur- und pH-Wert-Messungen auf der Haut mittels eines nicht-photonischen Messprinzips erhalten werden.

Die Überwachung diverser Vitalparameter erlangt zunehmend Bedeutung im Fitnesssektor. Es konnte bspw. gezeigt werden, dass übergewichtige Patienten mit einem personalisierten Ernährungsprogramm mehr an Gewicht verlieren, wenn ihr Ernährungsplan an den gemessenen Sauerstoffverbrauch (Detektion und Analyse von Stoffwechselparametern; Metabolic) angepasst wird.

Ein weiterer wichtiger Anwendungsbereich liegt in der Medizintechnik. Mittels des erfindungsgemäßen Sensormoduls können vorteilhaft Stoffwechselkrankheiten, wie Diabetes, detektiert werden, oder enzymatische Störungen oder Störungen der Darmflora kontinuierlich über einen längeren Zeitraum untersucht werden.

Zur Erhöhung der Patientensicherheit, z. B. während Operationen oder bei der Intensivüberwachung, ist der Erhalt von repräsentativen Proben vom inneren partiellen Gasaustausch der Lungen sehr wünschenswert. Das erfindungsgemäße Sensormodul bietet z. B. die Kombination eines ultraschnellen Sauerstoff- und/oder Kohlenstoffdioxid-Partialdrucksensors (pO₂ und/oder pCO₂) mit der Messung des Durchflusses der Atemgase an der Atemwegsöffnung und liefert damit diese höchst erwünschten und entscheidenden Informationen, die dann bspw. zur Optimierung der Beatmungsgeräteeinstellungen bei Intensivpatienten und Patienten in Anästhesie eingesetzt werden kann.

CO₂ und O₂ sind Gase, die leicht durch Körper- und Hautgewebe diffundieren und dadurch anhand eines entsprechenden nicht-invasiven Sensors, der auf der Hautoberfläche angebracht wird, gemessen werden können. Für transkutane Messungen kann das erfindungsgemäße Sensormodul an einem auf die Haut applizierbaren Träger angeordnet sein.

Das erfindungsgemäße Sensormodul kann an einer Zahnspange, einer Nasenklemme oder einem Beißschutz angeordnet sein.

Oder das Sensormodul kann in einem Beatmungsgerät und/oder Anästhesiegerät und/oder Lungenfunktionsgerät integriert sein.

Des Weiteren kann das erfindungsgemäße Sensormodul an einem Sammelgefäß für Urin- und Stuhlproben sowie Blutproben angeordnet sein.

Die Anordnung umfasst im Sinne dieser Anwendung jeweils auch Ausführungsformen, in denen das Sensormodul für das Medium zugänglich in die oben genannten Hilfsmittel eingebettet ist.

Bei der Verwendung des erfindungsgemäßen Sensormoduls zur Überwachung von Vitalparametern kann das Sensormodul vorteilhaft in Tier oder Mensch implantiert werden.

Das erfindungsgemäße Sensormodul kann in einer weiteren Ausführungsform als Kapsel verpackt zum Verschlucken ausgeführt sein. Dies ermöglicht ein besseres Durchgleiten des Magen-Darm-Trakts.

Die erfindungsgemäßen Verwendungen des erfindungsgemäßen Sensormoduls bieten mehrere Vorteile, von denen im Folgenden einige aufgeführt werden.

Aufgrund der geringen Anschaffungskosten und der Möglichkeit zur werksseitigen Kalibrierung/Justage sowie Sterilisierung kann das erfindungsgemäße Sensormodul als Einwegartikel ausgelegt sein und mit den Urin-, Blut- oder Stuhlproben entsorgt werden, was auch das Risiko von Kreuzkontaminationen minimiert.

Durch die multiparametrische Analyse des Mediums ist eine parallele Echtzeit- bzw. Online-Überwachung verschiedener Vitalparameter möglich.

Die erfindungsgemäßen Verwendungen können non-invasiv gestaltet werden; es wird kein Analyt verbraucht.

Gegenüber den herkömmlichen elektrochemischen Sauerstoffsensoren ist das der Erfindung zugrunde liegende Sensormodul umweltfreundlicher, da die aktuell in der O₂-Sensorik zum Einsatz kommenden hohen Anteile von Blei vermieden werden.

Aufgrund ultraschneller Ansprechzeiten (<50 ms rise time von 10 %-90 %) und geringer Baugröße (< 1 ml, < 25 g), des geringen Energieverbrauches, der geringen Verlustwärme kann die Überwachung von Vitalparametern beispielsweise direkt im Hauptstrom der Atmung erfolgen, ohne die Patientensicherheit zu gefährden oder bei brennbaren bzw. explosiven Analyten, Reinigungsmitteln oder Anästhetika eine Zündgefahr darzustellen (Explosionsschutz, ATEX). Das Sensormodul kann sterilisiert werden, z. B. durch Gammastrahlung, Plasma-Sterilisation, Autoklavierung, sterilisierende Reinigungsmittel.

Das erfindungsgemäße Sensormodul kann autonom energieversorgt ausgeführt werden. Es bietet die Möglichkeit einer Funkkommunikation, ist ultra-kompakt und hat geringste Verlustleistungen, kann photonische- und nicht-photonische sensorische Parameter detektieren, besitzt integrierte Methoden zur Referenzierung, und die Funktionsschicht kann gleichzeitig Teil der Verkapslung zum Medium darstellen, was die Miniaturisierung erleichtert

Die Erfindung ist nicht auf die dargestellten und beschriebenen Ausführungsformen beschränkt, sondern umfasst auch alle im Sinne der Erfindung gleich wirkenden Ausführungsformen. Ferner ist die Erfindung auch nicht auf die speziell beschriebenen Merkmalskombinationen beschränkt, sondern kann auch durch jede beliebige andere Kombination von bestimmten Merkmalen aller insgesamt offenbarten Einzelmerkmale definiert sein, sofern sich die Einzelmerkmale nicht gegenseitig ausschließen, oder eine spezifische Kombination von Einzelmerkmalen nicht explizit ausgeschlossen ist. Die Erfindung ist nur durch die beigefügten Ansprüche beschränkt.

Die Erfindung wird im Folgenden anhand von Ausführungsbeispielen erläutert, ohne auf diese beschränkt zu sein.

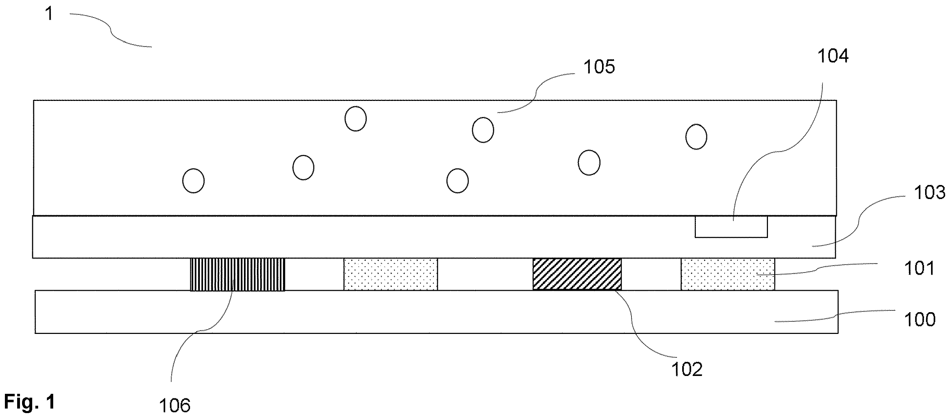

Dabei zeigt
Tabelle 1 Beispiele für photonische Messprinzipien zur Bestimmung von verschiedenen Eigenschaften eines Mediums und Beispiele für die sensorisch aktive Komponente;
Tabelle 2 Beispiele für nicht-photonische Messprinzipien zur Bestimmung von verschiedenen Eigenschaften eines Mediums und Beispiele für Vorrichtungen zum Messen der Eigenschaft Fig. 1 eine schematische Seitenansicht eines erfindungsgemäßen Sensormoduls zur multiparametrischen Analyse eines Mediums;
Fig. 2 eine schematische Seitenansicht einer Ausführungsform eines erfindungsgemäßen Sensormoduls zur multiparametrischen Analyse eines Mediums;
Fig. 3 ein erfindungsgemäßes Sensormodul in einer Fermentationskammer.

**Tabelle 1: Beispiele für photonische Messprinzipien**

| **Eigenschaft des Mediums** | **Messprinzip/ Messmethode** | **Sensorisch aktive Komponente** |
|---|---|---|
| Gelöster Sauerstoff | Photonisch/ Fluoreszenzmessung | organischer Farbstoff (z.B. PtTFPL in PSAN oder PtTFPP in Polystyrol) |
| pH-Wert | Photonisch/ Fluoreszenzmessung | organischer Farbstoff (z.B. HPTS, Seminaphtharhodafluor (SNARF), Hydroxycoumarin) |
| Temperatur | Photonisch/ Fluoreszenzmessung | organischer Farbstoff (z.B. Rhodamin, oder Eu(tta)₃(dpbt) in PVC) |
| Gelöstes CO₂ | Photonisch/ Fluoreszenzmessung | organischer Farbstoff (z.B. HPTS) in Kombination mit einem gaspermeablen und Protonen-undurchlässigen Polymer (z.B. Teflon, Silicon oder Polytetrafluoroethylen (PTFE)) |
| Glukose | Photonisch/ Fluoreszenzmessung | organischer Farbstoff (z.B. PtTFPL oder PtTFPP) in Kombination mit GlukoseOxidase-Enzym (Glukoseoxidase) |
| Lactat | Photonisch/ Fluoreszenzmessung | organischer Farbstoff (z.B. PtTFPL oder PtTFPP) in Kombination mit Laktatoxidase |
| Druck | Photonisch/ Fluoreszenzmessung | organischer Farbstoff (z.B. PtTFPL oder PtTFPP in FIB) |
| Toxinmessung | Photonisch/ Fluoreszenzmessung | GFP-Protein im GFP-Gen |
| Toxinmessung | Photonisch/ SPR-Messung | lonenkanal auf dünner, noblem Metallschicht / -Schichtsystem |
| Brechzahl | Photonisch/ SPR-Messung | Dünne, noble Metallschicht/-Schichtsystem (z.B. Ag, Au) |
| Wasserstoff | Photonisch/ SPR-Messung | Noble Metallschicht / Schichtsystem (z. B. Palladium-Schicht) |
| pH | Photonisch/ kolorimetrische Messung | Kolorimetrische Indikatoren (colorimetric indicators; z. B. Bromphenolblau [bromocresol purple]) |
| Kohlenstoffmonoxid | Photonisch/ kolorimetrische Messung | Kolorimetrische Indikatoren (z.B. binuklearer Rhodiumkomplex) |
| Stickstoffdioxid | Photonisch/ kolorimetrische Messung | Kolorimetrische Indikatoren (z.B. N,N,N',N' Tetramethyl-pphenylendiamin; Chinone) |
| Ammonium | Photonisch/ ratiometrische Fluoreszenzmessung | organischer Farbstoff (z.B. Oxazine 170 Perchlorat für Ammonium, Oxazine170 Perchlorate-Ethyl Cellulose (O17-EC) Membran mit aluminiumhaltigen Verbindungen zur Nitratmessung) |
| SpO2 | Photonisch/ ratiometirische Absorptionsmessung | 2-Wellenlängen-Reflexpulsoximetrie |

**Tabelle 2: Beispiele für nicht-photonische Messprinzipien**

| **Eigenschaft des Mediums** | **Messprinzip/ Messmethode** | **Vorrichtung** |
|---|---|---|
| pH-Wert | Nicht-photonisch/ elektrochemische Messung | Elektrodenanordnung mit z.B. MetalloxidSchicht als Arbeitselektrode (z.B. Iridium-Oxid, Ruthenium-Oxid), Silber/Silberchlorid als Referenzelektrode); ISFET |
| Temperatur | Nicht-photonisch/ Bandabstandsreferenz | Brokaw-Zelle Platin-Dickschichtsensor R-on-CMOS |
| Zellwachstum / Zelltod | Nicht-photonisch/ Impedanzmessverfahren | Interdigitale Elektrodenstruktur |
| Gelöstes CO₂ | Nicht-photonisch/ potentiometrisches Messprinzip | Elektrodenanordnung in Kombination mit einem gaspermeablen und Protonen-undurchlässigen Polymer (z.B. Teflon, Silicon oder Polytetrafluoroethylen (PTFE)) |
| Glukose | Nicht-photonisch/ amperometrische Messung | 2- oder 3-Elektrodenanordnung in Kombination mit Enzym Glukoseoxidase |
| Laktat | Nicht-photonisch/ amperometrische Messung | 2- bzw. 3-Elektrodenanordnung in Kombination mit Enzym Laktatoxidase |
| Druck | Nicht-photonisch/ | Dünnfilmsensor, Dickfilmsensor, piezoresistiver Sensor, MEMS-Sensor |

Bei der Kombination des mindestens einen photonischen Messprinzips mit einer Vorrichtung zur Temperaturmessung kann der Vorteil des erfindungsgemäßen Sensormoduls, dass der Messort der gewünschten Parameter vergleichsweise frei gewählt werden kann, besonders günstig ausgenutzt werden, indem z. B. ein R-on-CMOS-Sensor direkt neben einer Detektionsstrukturfür Fluoreszenzlicht angeordnet sein kann, sodass ohne große Verzögerung oder Übertragungskennlinie die Korrekturgröße "Temperatur" deszugehörigen Fluoreszenzfarbstoffes detektiert werden kann.

Fig. 1 zeigt schematisch eine Seitenansicht eines erfindungsgemäßen Sensormoduls 1 zur multiparametrischen Analyse eines Mediums 105. Das Sensormodul 1 weist mindestens einen organischen Lichtemitter zur Aussendung eines ersten photonischen Signals 101 und mindestens eine Vorrichtung zur Detektion photonischer Signale 102 auf. Der organische Lichtemitter 101 und die Vorrichtung zur Detektion photonischer Signale 102 sind dabei monolithisch auf einem Halbleitersubstrat 100 ausgebildet, welches vorteilhaft als CMOS-Halbleitersubstrat ausgebildet ist. Weiterhin weist das Sensormodul 1 mindestens ein Funktionsschichtsystem 103 auf, welches den mindestens einen organischen Lichtemitter 101 und/oder die mindestens eine Vorrichtung zur Detektion photonischer Signale 102 überdeckt und in Kontakt mit dem Medium 105 steht. Das Funktionsschichtsystem 103 weist mindestens einen aktiven Bereich 104 auf. Das Sensormodul 1 weist ein Bauelement zur Bestimmung einer zweiten Eigenschaft mittels eines nicht photonischen Messprinzips 106 auf, welches auf dem Halbleitersubstrat 100 angeordnet ist.

Weiterhin weist das Sensormodul 1 vorteilhaft mindestens eine Vorrichtung zum Speichern von Daten und/oder zum Auswerten und Beeinflussen von Daten und/oder zur Übertragung von Daten und/oder zur Kommunikation und/oder mindestens eine Vorrichtung zur Ansteuerung und/oder zur Modulation/Demodulation des mindestens einen organischen Lichtemitters und/oder der mindestens einen Vorrichtung zur Detektion photonischer Signale 107 auf. Weiterhin weist das Sensormodul 1 vorteilhaft eine Vorrichtung zur Bereitstellung der für den Betrieb des Sensormoduls 1 aufzuwendenden elektrischen Energie 108 auf.

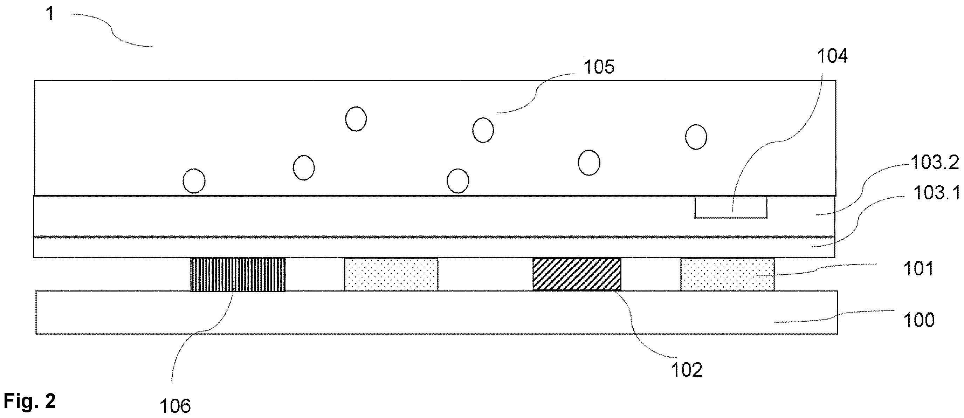

Fig. 2 zeigt eine schematische Seitenansicht einer Ausführungsform des erfindungsgemäßen Sensormoduls 1 zur multiparametrischen Analyse eines Mediums 105. Das Sensormodul 1 entspricht dem Sensormodul aus Fig. 1. Das Funktionsschichtsystem 103 weist einen Funktionsschichtträger 103.1 und eine Funktionsschicht 103.2 auf. Der Funktionsschichtträger 103.1 kann als optische Linse oder als optisches Linsenarray oder als optischer Filter oder als optisches Gitter oder als Kombination aus den vorbenannten ausgebildet sein.

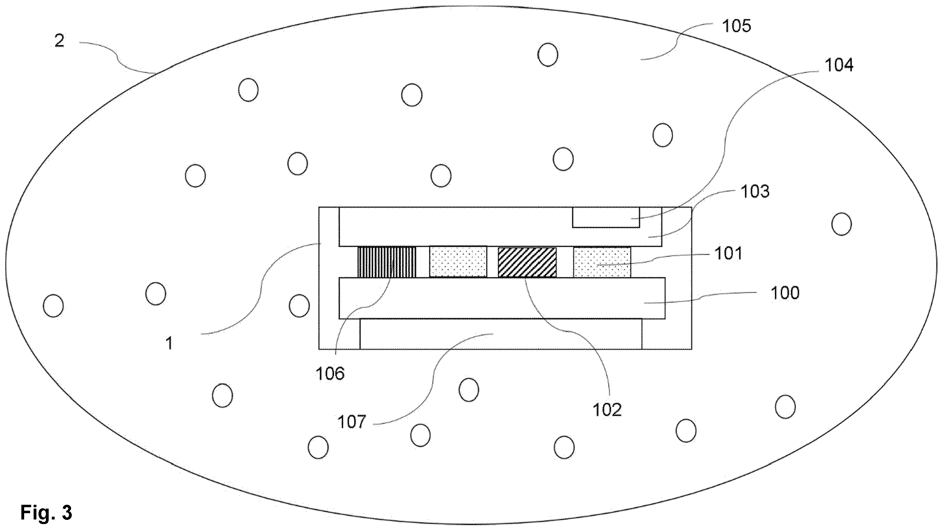

Fig. 3 zeigt schematisch ein Sensormodul 1 in einem Fermentationsreaktor 2, die mit einem Medium 105 gefüllt ist. Das Sensormodul 1 entspricht dem in Fig. 1 dargestellten Sensormodul 1. Erkennbar ist, dass das Funktionsschichtsystem 103 in Kontakt mit dem Medium 105 steht und den organischen Lichtemitter 101 und die Vorrichtung zur Detektion photonischer Signale 102 gegenüber dem Medium 105 abschirmt. Das Sensormodul 1 weist mindestens eine Vorrichtung zum Speichern von Daten und/oder zum Auswerten und Beeinflussen von Daten und/oder zur Übertragung von Daten und/oder zur Kommunikation 107 auf. Weiterhin kann das Sensormodul 1 mindestens eine Vorrichtung zur Ansteuerung und/oder zur Modulation/Demodulation des mindestens einen organischen Lichtemitters und/oder der mindestens einen Vorrichtung zur Detektion photonischer Signale (nicht dargestellt) aufweisen. Das Sensormodul 1 kann weiterhin eine Vorrichtung zur Bereitstellung der für den Betrieb des Sensormoduls aufzuwendenden elektrischen Energie aufweisen (nicht dargestellt).

### Bezugszeichen

- 1: Sensormodul
- 100: Halbleitersubstrat
- 101: Organischer Lichtemitter
- 102: Vorrichtung zur Detektion photonischer Signale
- 103: Funktionsschichtsystem
- 103.1: Funktionsschichtträger
- 103.2: Funktionsschicht
- 104: Aktiver Bereich
- 105: Medium
- 106: Bauelement zur Bestimmung zumindest einer zweiten Eigenschaft
- 107: Vorrichtung zum Speichern von Daten und/oder zum Auswerten und Beeinflussen von Daten und/oder zur Übertragung von Daten und/oder zur Kommunikation
- 2: Fermentationsreaktor

### Zitierte Nicht-Patentliteratur

[Krujatz2016] Krujatz F. et al "Exploiting the Potential of OLED-Based Photo-Organic Sensors for Biotechnological Applications"; Chem Sci J 2016 Vol. 7, Issue 3, 1000134, DOI: 10.4172/2150-3494.1000134

## Patentansprüche

1. Sensormodul (1) zur multiparametrischen Analyse eines Mediums (105), aufweisend
- mindestens einen organischen Lichtemitter (101) zur Aussendung eines ersten photonischen Signals und
- mindestens eine Vorrichtung zur Detektion photonischer Signale (102),
- mindestens ein Funktionsschichtsystem (103), das den mindestens einen organischen Lichtemitter (101) und/oder die mindestens eine Vorrichtung zur Detektion photonischer Signale (102) zumindest teilweise überdeckt und in Kontakt mit dem Medium (105) ist, wobei das Funktionsschichtsystem (103) so ausgebildet ist, dass es mindestens einen aktiven Bereich (104) aufweist, der mindestens eine Eigenschaft besitzt, die durch mindestens eine Eigenschaft des Mediums (105) beeinflussbar ist, und wobei der mindestens eine aktive Bereich (104) des Funktionsschichtsystems (103) mittels des mindestens einen organischen Lichtemitters (101) photonisch anregbar ist und ein zweites photonisches Signal aussendet, das mittels der mindestens einen Vorrichtung zur Detektion photonischer Signale (102) detektiert wird, wobei das zweite photonische Signal Informationen über die mindestens eine Eigenschaft des Mediums (105) enthält,
**dadurch gekennzeichnet, dass**
∘ der mindestens eine organische Lichtemitter (101) und die mindestens eine Vorrichtung zur Detektion photonischer Signale (102) monolithisch auf einem Halbleitersubstrat (100) oder in einem Halbleitersubstrat (100) ausgebildet sind und
∘ dass in dem Halbleitersubstrat mindestens eine Vorrichtung zum Speichern von Daten und/oder zum Auswerten und Beeinflussen von Daten und/oder zur Übertragung von Daten und/oder mindestens eine Vorrichtung zur Ansteuerung und/oder zur Modulation/Demodulation des mindestens einen organischen Lichtemitters und/oder der mindestens einen Vorrichtung zur Detektion photonischer Signale ausgebildet sind, und
∘ dass das Halbleitersubstrat (100) so ausgebildet ist, dass zumindest eine zweite Eigenschaft mittels eines nicht-photonischen Messprinzips ermittelbar ist oder das Sensormodul mindestens ein Bauelement (106) zur Bestimmung zumindest einer zweiten Eigenschaft mittels eines nicht-photonischen Messprinzips aufweist, das auf dem Halbleitersubstrat (100) angeordnet ist.

2. Sensormodul (1) nach Anspruch 1, **dadurch gekennzeichnet, dass** das Halbleitersubstrat (100) als CMOS ausgebildet ist.

3. Sensormodul (1) nach Anspruch 1 oder 2, **dadurch gekennzeichnet, dass** das Halbleitersubstrat (100) umfasst:
i.) mindestens eine Vorrichtung zur Durchführung einer elektrochemischen Messung welche bevorzugt eine Electrode-on-CMOS- oder ISFET- oder ChemFET- oder ENFET- oder pH-FET oder Solid-state-electrolyte-Struktur oder eine Kombination aus den vorbenannten umfasst, oder
ii.) mindestens eine Vorrichtung zur Durchführung einer Temperaturmessung welche bevorzugt einen R-on-CMOS- oder einen Thermoelement- oder einen Halbleiter-Sensor oder eine Kombination aus den vorbenannten umfasst, oder
iii.) mindestens eine Vorrichtung zur Durchführung einer Impedanzmessung, welche bevorzugt eine interdigitale Elektrodenstruktur umfasst, oder
iv.) mindestens eine Vorrichtung zur Durchführung einer Magnetfeldmessung, welche bevorzugt einen Hallsensor umfasst, oder
v.) mindestens eine Vorrichtung zur Durchführung einer Rückstreumessung oder
vi.) mindestens eine Vorrichtung zur Durchführung einer Durchflussmessung oder
vii.) mindestens eine Vorrichtung zur Durchführung einer Strömungsgeschwindigkeitsmessung, welche bevorzugt das Prinzip eines Hitzdrahtanemometers umfasst, oder
viii.) mindestens eine Vorrichtung zur Durchführung einer Wärmestrommessung, welche bevorzugt einen Thermosäulensensor umfasst, oder
ix.) mindestens eine Vorrichtung zur Durchführung einer Druckmessung, welche bevorzugt einen Dünnfilm- oder Dickfilm- oder piezoresistiven oder MEMS-Sensor oder eine Kombination aus den vorbenannten umfasst, oder
x.) eine Kombination aus den vorbenannten.

4. Sensormodul (1) nach einem der Ansprüche 1 bis 3, **dadurch gekennzeichnet, dass** das Sensormodul (1) mehrere organische Lichtemitter (101) aufweist, die an definierbaren Positionen räumlich voneinander getrennt anordenbar sind und die jeweils ein erstes photonisches Signal mit voneinander verschiedener Wellenlänge aussenden oder jeweils ein erstes photonisches Signal mit gleicher Wellenlänge aussenden.

5. Sensormodul (1) nach einem der Ansprüche 1 bis 4, **dadurch gekennzeichnet, dass** das Sensormodul (1) mehr als eine Vorrichtung zur Detektion photonischer Signale (102) aufweist, die an definierbaren Positionen räumlich voneinander getrennt anordenbar sind, wobei die Vorrichtungen zur Detektion photonischer Signale (102) so ausgebildet sind, dass sie voneinander verschiedene spektrale Sensitivitätsbereiche aufweisen, so dass sie jeweils ein zweites photonisches Signal mit voneinander verschiedener Wellenlänge detektieren, oder wobei die Vorrichtungen zur Detektion photonischer Signale (102) so ausgebildet sind, dass sie die gleichen oder sich überlappende Sensitivitätsbereiche aufweisen, wobei eine Zuordnung des jeweils detektierten zweiten photonischen Signals zum Ort seiner Aussendung über unterscheidbare numerische Aperturen erfolgt

6. Sensormodul (1) nach den einem der Ansprüche 1 bis 5, **dadurch gekennzeichnet, dass** mindestens eine Vorrichtung zur Detektion photonischer Signale (102) unmittelbar unterhalb mindestens eines organischen Lichtemitters (101) angeordnet ist.

7. Sensormodul (1) nach einem der Ansprüche 1 bis 6, **dadurch gekennzeichnet, dass** das Funktionsschichtsystem (103) mehr als einen aktiven Bereich (104) aufweist, wobei die aktiven Bereiche (104) räumlich voneinander getrennt angeordnet sind und bevorzugt so ausgebildet sind, dass die von getrennten aktiven Bereichen (104) ausgesandten zweiten photonischen Signale Informationen über verschiedene Eigenschaften des Mediums (105) enthalten.

8. Sensormodul (1) nach einem der Ansprüche 1 bis 7, **dadurch gekennzeichnet, dass** das Funktionsschichtsystem (103) mindestens eine Funktionsschicht (103.2) umfasst, die auf einem Funktionsschichtträger (103.1) angeordnet ist,
wobei der Funktionsschichtträger (103.1) bevorzugt als optische Linse oder als optisches Linsenarray oder als optischer Filter oder als optisches Gitter oder als Kombination aus den vorbenannten ausgebildet ist.

9. Sensormodul (1) nach einem der Ansprüche 1 bis 8, **dadurch gekennzeichnet, dass** das Sensormodul (1) zusätzlich aufweist:
i. mindestens eine Vorrichtung zum Speichern von Daten und/oder zum Auswerten und Beeinflussen von Daten und/oder zur Übertragung von Daten und/oder zur Kommunikation (107) und/oder
ii. eine Vorrichtung zur Ansteuerung und/oder zur Modulation/Demodulation des mindestens einen organischen Lichtemitters (101) und/oder der mindestens einen Vorrichtung zur Detektion photonischer Signale (102).

10. Sensormodul (1) nach Anspruch 9, **dadurch gekennzeichnet, dass** die mindestens eine Vorrichtung (107) monolithisch im Halbleitersubstrat (100) ausgebildet ist.

11. Sensormodul (1) nach einem der Ansprüche 1 bis 10, **dadurch gekennzeichnet, dass** das Sensormodul (1) mindestens eine Vorrichtung zur Bereitstellung der für den Betrieb des Sensormoduls (1) aufzuwendenden elektrischen Energie aufweist.

12. Sensormodul (1) nach einem der Ansprüche 1 bis 11, **dadurch gekennzeichnet, dass** am Sensormodul (1) mindestens eine aktorische Komponente angeordnet ist.

13. Verwendung des Sensormoduls (1) nach einem der Ansprüche 1 bis 12 in einem Lab-on-a-chip-System oder zur Fermentationsüberwachung, insbesondere in einem Einweg-Fermentationsbeutel.

14. Verwendung des Sensormoduls (1) nach einem der Ansprüche 1 bis 12 zur Überwachung von Vitalparametern.

15. Verwendung nach Anspruch 14, **dadurch gekennzeichnet, dass**
a.) das Sensormodul (1) in einem auf die Haut applizierbaren Träger angeordnet ist, oder
b.) das Sensormodul (1) an einer Zahnspange, einer Nasenklemme oder einem Beißschutz angeordnet ist, oder
c.) das Sensormodul (1) in einem Beatmungsgerät oder Anästhesiegerät oder Lungenfunktionsgerät integriert ist, oder
d.) das Sensormodul (1) in einem Sammelgefäß für Urin- oder Stuhl- oder Blutproben angeordnet ist, oder e.) das Sensormodul (1) implantierbar ausgeführt ist, oder .
f.) das Sensormodul (1) in einer verschluckbaren Kapsel angeordnet ist.

## Claims

1. Sensor module (1) for multiparametric analysis of a medium (105), comprising
- at least one organic light emitter (101) for emitting a first photonic signal and
- at least one device for detecting photonic signals (102),
- at least one functional layer system (103) which at least partially covers the at least one organic light emitter (101) and/or the at least one device for detecting photonic signals (102) and is in contact with the medium (105), wherein the functional layer system (103) is designed so that it comprises at least one active region (104) having at least one property that can be influenced by at least one property of the medium (105),
and wherein the at least one active region (104) of the functional layer system (103) can be photonically excited by means of the at least one organic light emitter (101) and emits a second photonic signal which is detected by the at least one device for detecting photonic signals (102), the second photonic signal containing information about the at least one property of the medium (105),
**characterised in that**
∘ the at least one organic light emitter (101) and the at least one device for detecting photonic signals (102) are monolithically formed on a semiconductor substrate (100) or in a semiconductor substrate (100) and
∘ **in that** at least one device for storing data and/or for evaluating and influencing data and/or for transmitting data and/or at least one device for the control and/or for the modulation/demodulation of the at least one organic light emitter and/or the at least one device for detecting photonic signals are constructed in the semiconductor substrate, and
∘ **in that** the semiconductor substrate (100) is designed so that it is able to determine at least one second property by means of a non-photonic measurement principle or the sensor module has at least one component (106) for determining at least one second property by means of a non-photonic measurement principle which is arranged on the semiconductor substrate (100).

2. Sensor module (1) according to claim 1, **characterised in that** the semiconductor substrate (100) is designed as a CMOS.

3. Sensor module (1) according to claim 1 or 2, **characterised in that** the semiconductor substrate (100) comprises:
i.) at least one device for performing an electrochemical measurement, which preferably comprises an electrode-on-CMOS or ISFET or ChemFET or ENFET or pH-FET or solid-state electrolyte structure or a combination of the aforementioned, or
ii.) at least one device for performing temperature measurement which preferably comprises an R-on-CMOS sensor or a thermocouple sensor or a semiconductor sensor or a combination of the aforementioned, or
iii.) at least one device for performing an impedance measurement which preferably comprises an interdigital electrode structure, or
iv.) at least one device for performing a magnetic field measurement which preferably comprises a Hall sensor, or
v.) at least one device for performing a backscatter measurement, or
vi.) at least one device for performing a flow measurement, or
vii.) at least one device for performing a flow rate measurement which preferably comprises the principle of a hot wire anemometer, or
viii.) at least one device for performing a heat flow measurement which preferably comprises a thermopile sensor, or
ix.) at least one device for performing a pressure measurement which preferably comprises a thin-film sensor or a thick-film sensor or a piezoresistive sensor or a MEMS sensor or a combination of the aforementioned, or
x.) a combination of the aforementioned.

4. Sensor module (1) according to any one of claims 1 to 3, **characterised in that** the sensor module (1) has a plurality of organic light emitters (101) which can be arranged spatially separated from one another at definable positions and which each emit a first photonic signal having wavelengths that differ from one another or each emit a first photonic signal having the same wavelength.

5. Sensor module (1) according to any one of claims 1 to 4, **characterised in that** the sensor module (1) has more than one device for detecting photonic signals (102) which can be arranged spatially separated from one another at definable positions, wherein the devices for detecting photonic signals (102) are designed so as to have spectral sensitivity ranges that differ from one another, so that they each detect a second photonic signal having wavelengths that differ from one another, or wherein the devices for detecting photonic signals (102) are designed so as to have the same sensitivity ranges or overlapping sensitivity ranges, the assignment of the corresponding detected second photonic signal to the location of the emission thereof taking place via distinguishable numerical apertures.

6. Sensor module (1) according to any one of claims 1 to 5, **characterised in that** at least one device for detecting photonic signals (102) is arranged directly below at least one organic light emitter (101).

7. Sensor module (1) according to any one of claims 1 to 6, **characterised in that** the functional layer system (103) has more than one active region (104), the active regions (104) being arranged spatially separated from one another and are preferably designed so that the second photonic signals emitted by separate active regions (104) contain information about various properties of the medium (105).

8. Sensor module (1) according to any one of claims 1 to 7, **characterised in that** the functional layer system (103) comprises at least one functional layer (103.2) which is arranged on a functional layer carrier (103.1),
wherein the functional layer carrier (103.1) is preferably designed as an optical lens or as an optical lens array or as an optical filter or as an optical lattice or as a combination of the aforementioned.

9. Sensor module (1) according to any one of claims 1 to 8, **characterised in that** the sensor module (1) additionally comprises:
i. at least one device for storing data and/or for evaluating and influencing data and/or for transmitting data and/or for communication (107) and/or
ii. a device for the control and/or for the modulation/demodulation of the at least one organic light emitter (101) and/or the at least one device for detecting photonic signals (102).

10. Sensor module (1) according to claim 9, **characterised in that** the at least one device (107) is formed monolithically in the semiconductor substrate (100).

11. Sensor module (1) according to any one of claims 1 to 10, **characterised in that** the sensor module (1) comprises at least one device for providing the electrical energy to be expended for the operation of the sensor module (1).

12. Sensor module (1) according to any one of claims 1 to 11, **characterised in that** at least one actuator component is arranged on the sensor module (1).

13. Use of the sensor module (1) according to any one of claims 1 to 12 in a lab-on-a-chip system or for fermentation monitoring, in particular in a disposable fermentation bag.

14. Use of the sensor module (1) according to one of the claims 1 to 12 for monitoring vital parameters.

15. Use according to claim 14, **characterised in that**
a.) the sensor module (1) is arranged in a carrier which can be applied to the skin, or
b.) the sensor module (1) is arranged on a brace, a nose clip or a bite guard, or
c. ) the sensor module (1) is integrated in a respirator or anaesthesia device or pulmonary function device, or
d.) the sensor module (1) is arranged in a collection vessel for urine or faeces or blood samples, or e.) the sensor module (1) is designed to be implantable, or
f.) the sensor module (1) is arranged in an ingestible capsule.

## Revendications

1. Module de capteurs (1) pour l'analyse multiparamétrique d'un milieu (105), présentant
- au moins un émetteur de lumière organique (101) permettant d'émettre un premier signal photonique et
- au moins un dispositif permettant de détecter des signaux photoniques (102),
- au moins un système à couche fonctionnelle (103) qui recouvre au moins partiellement l'au moins un émetteur de lumière organique (101) et/ou l'au moins un dispositif permettant de détecter des signaux photoniques (102) et est en contact avec le milieu (105), dans lequel le système à couche fonctionnelle (103) est conçu de sorte qu'il présente au moins une zone active (104), laquelle possède au moins une propriété pouvant être influencée par au moins une propriété du milieu (105), et dans lequel l'au moins une zone active (104) du système à couche fonctionnelle (103) peut être excitée de manière photonique au moyen de l'au moins un émetteur de lumière organique (101) et émet un second signal photonique qui est détecté au moyen de l'au moins un dispositif permettant de détecter des signaux photoniques (102), dans lequel le second signal photonique contient des informations concernant l'au moins une propriété du milieu (105),
**caractérisé en ce que**
∘ l'au moins un émetteur de lumière organique (101) et l'au moins un dispositif permettant de détecter des signaux photoniques (102) sont conçus de manière monolithique sur un substrat semi-conducteur (100) ou dans un substrat semi-conducteur (100) et
∘ **qu'**au moins un dispositif permettant de stocker des données et/ou d'évaluer et d'influencer des données et/ou de transmettre des données et/ou au moins un dispositif permettant de commander et/ou de moduler/démoduler l'au moins un émetteur de lumière organique et/ou l'au moins un dispositif permettant de détecter des signaux photoniques sont conçus dans le substrat semi-conducteur, et
∘ **que** le substrat semi-conducteur (100) est conçu de sorte qu'au moins une seconde propriété peut être déterminée au moyen d'un principe de mesure non photonique ou le module de capteurs présente au moins un composant (106) permettant de déterminer au moins une seconde propriété au moyen d'un principe de mesure non photonique, lequel composant est disposé sur le substrat semi-conducteur (100).

2. Module de capteurs (1) selon la revendication 1, **caractérisé en ce que** le substrat semi-conducteur (100) est conçu en tant que CMOS.

3. Module de capteurs (1) selon la revendication 1 ou 2,
**caractérisé en ce que** le substrat semi-conducteur (100) comprend :
i.) au moins un dispositif permettant de réaliser une mesure électrochimique, lequel comprend de préférence une structure de type électrode sur CMOS ou ISFET ou ChemFET ou ENFET ou pH-FET ou électrolyte à l'état solide ou une combinaison des éléments susmentionnés, ou
ii.) au moins un dispositif permettant de réaliser une mesure de température, lequel comprend de préférence un capteur de type R sur CMOS ou un capteur à thermocouple ou un capteur à semi-conducteur ou une combinaison des éléments susmentionnés, ou
iii.) au moins un dispositif permettant de réaliser une mesure d'impédance, lequel comprend de préférence une structure d'électrodes interdigitale, ou
iv.) au moins un dispositif permettant de réaliser une mesure de champ magnétique, lequel comprend de préférence un capteur à effet Hall, ou
v.) au moins un dispositif permettant de réaliser une mesure de rétrodiffusion ou
vi.) au moins un dispositif permettant de réaliser une mesure de débit ou
vii.) au moins un dispositif permettant de réaliser une mesure de vitesse d'écoulement, lequel comprend de préférence le principe d'un anémomètre à fil chaud, ou
viii.) au moins un dispositif permettant de réaliser une mesure de flux thermique, lequel comprend de préférence un capteur à thermopile, ou
ix.) au moins un dispositif permettant de réaliser une mesure de pression, lequel comprend de préférence un capteur à couche mince ou à couche épaisse ou piézorésistif ou MEMS ou une combinaison des éléments susmentionnés, ou
x.) une combinaison des éléments susmentionnés.

4. Module de capteurs (1) selon l'une des revendications 1 à 3,
**caractérisé en ce que** le module de capteurs (1) présente plusieurs émetteurs de lumière organiques (101) qui peuvent être disposés dans des positions définissables de manière à être séparés spatialement les uns des autres et qui émettent respectivement un premier signal photonique de longueurs d'onde différentes les unes des autres ou émettent respectivement un premier signal photonique de même longueur d'onde.

5. Module de capteurs (1) selon l'une des revendications 1 à 4,
**caractérisé en ce que** le module de capteurs (1) présente plus d'un dispositif permettant de détecter des signaux photoniques (102) qui peuvent être disposés dans des positions définissables de manière à être séparés spatialement les uns des autres, dans lequel les dispositifs permettant de détecter des signaux photoniques (102) sont configurés de sorte qu'ils présentent des plages de sensibilité spectrales différentes les unes des autres, de sorte qu'ils détectent respectivement un second signal photonique de longueurs d'onde différentes les unes des autres, ou dans lequel les dispositifs permettant de détecter des signaux photoniques (102) sont configurés de sorte qu'ils présentent les mêmes plages de sensibilité ou des plages de sensibilité se chevauchant, dans lequel une attribution du second signal photonique respectivement détecté à l'emplacement de son émission est effectuée par l'intermédiaire d'ouvertures numériques pouvant être différenciées.

6. Module de capteurs (1) selon l'une des revendications 1 à 5,
**caractérisé en ce qu'**au moins un dispositif permettant de détecter des signaux photoniques (102) est disposé directement en dessous d'au moins un émetteur de lumière organique (101).

7. Module de capteurs (1) selon l'une des revendications 1 à 6,
**caractérisé en ce que** le système à couche fonctionnelle (103) présente plus d'une zone active (104), dans lequel les zones actives (104) sont disposées de manière à être séparées spatialement les unes des autres et sont de préférence configurées de sorte que les seconds signaux photoniques émis par des zones actives (104) séparées contiennent des informations concernant différentes propriétés du milieu (105).

8. Module de capteurs (1) selon l'une des revendications 1 à 7,
**caractérisé en ce que** le système à couche fonctionnelle (103) comprend au moins une couche fonctionnelle (103.2) qui est disposée sur un support de couche fonctionnelle (103.1),
dans lequel le support de couche fonctionnelle (103.1) est conçu de préférence en tant que lentille optique ou en tant que réseau de lentilles optique ou en tant que filtre optique ou en tant que réseau optique ou en tant que combinaison des éléments susmentionnés.

9. Module de capteurs (1) selon l'une des revendications 1 à 8,
**caractérisé en ce que** le module de capteurs (1) présente en outre :
i. au moins un dispositif permettant de stocker des données et/ou d'évaluer et d'influencer des données et/ou de transmettre des données et/ou de communiquer (107) et/ou
ii. un dispositif permettant de commander et/ou de moduler/démoduler l'au moins un émetteur de lumière organique (101) et/ou l'au moins un dispositif permettant de détecter des signaux photoniques (102).

10. Module de capteurs (1) selon la revendication 9, **caractérisé en ce que** l'au moins un dispositif (107) est conçu de manière monolithique dans le substrat semi-conducteur (100).

11. Module de capteurs (1) selon l'une des revendications 1 à 10, **caractérisé en ce que** le module de capteurs (1) présente au moins un dispositif permettant de fournir l'énergie électrique à utiliser pour le fonctionnement du module de capteurs (1).

12. Module de capteurs (1) selon l'une des revendications 1 à 11, **caractérisé en ce qu'**au moins un composant actionneur est disposé sur le module de capteurs (1).

13. Utilisation du module de capteurs (1) selon l'une des revendications 1 à 12
dans un système de type laboratoire sur puce ou pour la surveillance de la fermentation, en particulier dans un sac de fermentation jetable.

14. Utilisation du module de capteurs (1) selon l'une des revendications 1 à 12 pour la surveillance de paramètres vitaux.

15. Utilisation selon la revendication 14, **caractérisée en ce que**
a.) le module de capteurs (1) est disposé dans un support pouvant être appliqué sur la peau, ou
b.) le module de capteurs (1) est disposé sur un appareil dentaire, un pincenez ou un cale-dents, ou
c.) le module de capteurs (1) est intégré dans un respirateur ou un appareil d'anesthésie ou un appareil pour fonctions pulmonaires, ou
d.) le module de capteurs (1) est disposé dans un récipient de collecte pour des échantillons d'urine ou de selles ou de sang, ou e.) le module de capteurs (1) est conçu de manière à pouvoir être implanté, ou
f.) le module de capteurs (1) est disposé dans une capsule pouvant être avalée.
